# EUROPEAN PATENT APPLICATION

(11) **EP 3 461 411 A1**
(43) Date of publication of application: **03.04.2019**
(21) Application number: 17306291.0
(22) Date of filing: 28.09.2017
(51) Int. Cl.: A61B 6/00, A61B 34/10

(54) **AUGMENTED ANATOMICAL MAP**

(71) Applicant: Koninklijke Philips N.V., 5656 AE Eindhoven (NL)
(72) Inventor: DEN HARTOG, Markus Johannes Harmen, 5656 AE Eindhoven (NL); OLIVAN BESCOS, Javier, 5656 AE Eindhoven (NL); AUVRAY, Vincent Maurice André, 5656 AE Eindhoven (NL); FLORENT, Raoul, 5656 AE Eindhoven (NL); BULLENS, Roland Wilhelmus Maria B, 5656 AE Eindhoven (NL)
(74) Representative: de Haan, Poul Erik

(57) **Abstract**

The present invention relates to a method of generating an augmented anatomical map of an object, a corresponding device and system. The augmented anatomical map of the object is provided during an interventional procedure.

## Description

### FIELD OF THE INVENTION

The present invention relates to a method for augmenting an anatomical map of an object and an according device and system.

### BACKGROUND OF THE INVENTION

Percutaneous coronary interventions (PCI) are non-surgical procedures used to treat narrowing of the coronary arteries of the heart of a human being. In a typical PCI procedure, the interventionist will first acquire one or more angiograms to diagnose the disease, will then navigate towards the diseases area, perform additional imaging (IVUS, OCT), pre-dilate the occlusion and then place a stent. After which final angiograms are made to evaluate the result.

During PCI procedures, the exact location of different interventional actions has to be remembered by the clinician, while relevant landmarks are often invisible. The knowledge of the position of previous actions could be crucial for a successful treatment. For example, the part of the vessels that are pre-dilated, need to be fully covered by one or more stents.

### SUMMARY OF THE INVENTION

There may thus be a need to provide a device and a method so that the position of different previous interventional actions is provided to the interventionist in an appropriate manner.

The object of the present invention is solved by the subject-matter of the independent claims; further embodiments are incorporated in the dependent claims. It should be noted that the following described aspects of the invention also apply for the method, the device, the system and vice versa.

There are interventional procedures, e.g. a PCI procedure, which require accurate, detailed and/or exact information concerning the location of different previous interventional actions. Such location must be remembered by the clinician, a task that is all the more tedious as natural reference points (landmarks) on the vessels are invisible, sometimes even with the assistance of injection of contrast agent. The position of previous actions is often crucial for a successful treatment. For instance, a ballooned area must always be fully stented to prevent restenosis. During a complex case (multi vessel disease, Chronic Total Occlusion (CTO), bifurcations), it can sometimes be difficult to remember all ballooned places, which may lead to one or more ballooned areas not stented.

Also, the placement of more than one stent, in which case the secondly placed device needs to be accurately positioned with respect to the first one, requires an accurate or exact information about the location of the first stent.

According to an aspect of the present invention, a device of generating an augmented anatomical map of an object is provided. The device comprises a storage unit, configured for storing a reference anatomical map of the object and a processing unit. In an example, the reference anatomical map may be a previously augmented anatomical map of the object. The processing unit is configured to detect one or more events within a space of intervention, wherein the space of intervention is defined according to image data of the object; to translate a position of the event into the reference anatomical map; to augment the reference anatomical map by adding information representing the event; and to provide the augmented anatomical map of the object. The augmented anatomical map of the object is provided during or after an interventional procedure.

According to an example, the storage unit is provided for storing multiple reference anatomical maps, and the processing unit is configured to select a reference anatomical map of the object from the provided multiple reference anatomical maps. According to another aspect of the present invention, a system of generating an augmented anatomical map of an object is provided. The system comprises at least one imaging unit, a displaying unit and a device for augmenting an anatomical map of the object, wherein the augmented anatomical map is provided during an interventional procedure. In an example, the system comprises at least one interventional imaging system. Such interventional imaging system is configured to perform at least one of balloon inflations, a stent placement, functional measurements, intravascular ultrasound (IVUS), optical coherence tomography (OCT), quantitative coronary angiography (QCA), needle ablation, needle biopsy, beads injection, and the like. The interventional device can comprise several different interventional imaging systems.

According to an example, the at least one imaging unit is configured to generate at least an X-ray image. In an example, the imaging unit is configured to generate an angiogram or a fluoroscopic image.

The displaying unit may comprise one or more display devices. The augmented anatomical map is provided to the display unit for display on the one or more display device.

According to the present invention, a method of generating an augmented anatomical map of an object is provided. The method comprises: detecting one or more events within a space of intervention, wherein the space of intervention is defined according to image data of the object; translating a position of the event into the reference anatomical map; augmenting the reference anatomical map by adding information representing the event; and providing the augmented anatomical map of the object. The augmented anatomical map of the object is provided during or after an interventional procedure.

During the procedure, interventions / actions and their location / position are identified and recorded. For example, a balloon inflation event and its associated position might be reliably determined in a live fluoroscopy image. In another example, the location of a fractional flow reserve (FFR) measurement may be derived from pullback data including a start position and pullback velocity. Then, the determined event information is translated to the reference map, on which the positions and actions are marked. For this purpose, an accurate registration between the live image and the reference map may be required. This can be achieved by using salient points that are visible in both the reference as the live images and estimating the position based on the relative positions of the event location and the salient points. Motion compensation, either affine or non-rigid, may be required to compensate for motion due to 3D-2D projection errors, geometry movement, and/or patient/organ movement/deformation. Thus, a method is provided, so that for example previously inflated balloon locations are visualized on a reference angiogram or cardiac roadmap to create an anatomical map with event history. The invention is described with reference to PCI interventions, however it is also applicable to other interventions, where location of an event can be assessed and related to a map of the anatomy of the object.

A reference image or reference sequence of the object may be utilized as reference anatomical map. The reference anatomical map might be acquired pre-operational, i.e. before the actual intervention is performed. The reference anatomical map can be a previous augmented anatomical map. Or, the reference anatomical map is acquired at the start of the intervention procedure. Thus, the actual vasculature of the organ along with its events can be provided. The reference anatomical map can also be an atlas, i.e. constructed by combining a large number of anatomies or different patients into a single reference map.

The reference anatomical map of the object comprises the vasculature or other distinct relevant features of the object. The object can be a human organ of a patient, for example, such as a human heart, a liver, a brain, arms, legs, etc. In other words, a reference anatomical map providing the vessels or structure(s) of interest of the organ is selected. In some examples, the reference anatomical map can be extracted from a pre-intervention examination like an image received by computed tomography (CT), computed tomography angiography (CTA), angiogram or the like, or it can be an injected angiogram from the ongoing interventional procedure. In an example, the reference image/sequence is a synthetic map computed from different sources, e.g. different angiograms highlighting different vessels or a mixture of CT and angiograms to enrich the three-dimensional (3D) content with improved spatial resolution, etc. The choice of the reference volume(s) and/or image(s) for providing an anatomical map of the object can either be performed by the user, or it can be done automatically. In the case of automatic selection, dedicated selection criteria are provided in order to select the most relevant volume(s) / image(s). Such dedicated selection criteria comprise quantifying the quality of the vasculature visibility, for instance.

An event is detected within a space of intervention. According to an example, the space of intervention comprises the duration of a single intervention or multiple interventions, e.g. over a large period of time (days, month or even years). An event can be any interaction or change with a distinct time span, that can be related to a distinct part of the anatomical map. In some examples, the event can be an interaction or a location such as balloon inflations, a stent placement, functional measurements, intravascular ultrasound (IVUS), optical coherence tomography (OCT), quantitative coronary angiography (QCA), needle ablation, needle biopsy, beads injection, and the like.

The position of the detected event is translated into the reference anatomical map. The event can be translated along with detection in the image (e.g. initial device position). The accurate detection and position of the event over the live intervention image is translated into the reference anatomical map. In other words, the position of the event is translated into the referential of the reference anatomical map. The anatomical sub-vessel in which the event has taken place (mid LAD (Left Anterior Descending artery), second diagonal, etc.) is identified, and reported in the proper anatomical position over the reference anatomical map. This position is translated and marked in the reference anatomical map of the object.

The reference anatomical map of the object is augmented by adding information representing the event. The event, with the interaction history, can then be visualized on the pre-op angiogram of cardiac roadmap using several visualization methods, like overlays, colors, pictorials or actual images of the interaction. Next to these interaction visualizations, information could be presented, with characteristic of the interaction, like interaction order/time, functional measurements (FFR/IFR), stent-type etc. The location of the event that has been determined is highlighted over the reference anatomical map. It can be marked in reference colors, if the purpose is only to display where some events have taken place. If some measures were made (FFR, QCA, flow measurements, etc.), they can also be color-coded when reported over the reference anatomical map.

The augmented anatomical map of the object is provided for further processing and/or display. In other words, the augmented anatomical map comprises the information of the reference anatomical map of the object, comprising previous events, and also comprises current live events detected during the interventional procedure.

The augmented anatomical map is provided during an interventional procedure. Thus, an accurate, detailed and/or precise location of previous or actual live events is made visible and is provided to an interventionist.

The augmented anatomical map can also be used after the procedure, for reporting, making a case report or archiving for future interventions.

According to an example, the reference anatomical map is provided for further processing and/or display. The reference anatomical map can either be used as such, or it can be processed further to offer an improved vessel visibility. Such further processing (also referred to aspre-processing) can include background subtraction, volume reprojection from an optimal perspective, etc.

According to an example, the space of intervention comprises the duration of a single intervention or multiple interventions over a period of time. The space of intervention may comprise all events (previous and current) performed on the object during one or multiple interventions and is defined by the image data of the events. Thus, a history of the events throughout the interventions can be provided as augmented anatomical map.

According to an example, one or more events are automatically identified in an interventional image of the object. One or more events are automatically identified and recorded on the live image during the interventional procedure, either by image detection of the fluoroscopy/acquisition or by co-registration using other location specific information, e.g. automatic pullback. In other words, the event is detected and localized in the space of intervention. The event can also be referred to as event of interest, i.e. an event which knowledge supports the interventional procedure or in some cases is crucial for the interventional procedure. The event can also be a custom tag, initiated by the physician and manually added to the map using a mouse, keyboard or other interaction device.

According to an example, one or more events are detected in a fluoroscopic image or in a sequence of fluoroscopic images of the object. A mode of detection is to detect an event in the fluoroscopic images that are acquired during the intervention. For instance, a dilated balloon can be characterized as a regular contrasted region lying between radiopaque markers. Detecting those structures, provides knowledge of the time an inflation has been performed, and where it was deployed in the image taken during the interventional procedure. The same approach could be followed to detect OCT, FFRs, IVUS etc. in fluoroscopic images.

According to an example, measurements performed during the interventional procedure are utilized for detecting the one or more events. Such measurements are performed during certain phases of the intervention. For instance, if an automatic FFR pullback is performed, the exact instant where it begins is known, as well as the pullback velocity.

According to an example, additional information or image processing techniques are used for aiding the detecting of the one or more events, or for calculating the location on the reference anatomical map. The additional information can be context specific. The image processing techniques are specialized. For example, if a physician starts an FFR measurement, the guide wire detected in the image can be assumed to be an FFR wire, even if it cannot be distinguished with a normal guide wire using image analysis alone. For stent detection, multiple X-ray images can be analyzed, when the stent might not be visible in a single image. For IVUS fluoroscopic images the pullback velocity may be used to estimate the location, even without continuous fluoroscopic images being acquired. For QCA the physician clicks on the target lesion to get a functional measurement.

According to an example, translating the one or more events involves a cardiac roadmapping technique. In a case where the selection has been performed over the interventional image, and where the reference anatomical map is an angiogram acquired from the same angulation, this process is known as cardiac roadmapping.

According to an example, the anatomical map is updated, using newly acquired images/information during the intervention. For example, after opening a Chronic Total occlusion (CTO), a new vessel may appear, previously invisible. Also, an event might deform the anatomy, such as placement of a flow diverter, coiling an aneurysm, etc. The update to the anatomical map can be stored and reviewed later.

According to another aspect a computer program element for controlling a device for augmenting an anatomical map of an object is provided which, when being executed by a processing unit, is adapted to perform the mentioned method steps. Also, according to another aspect, a computer readable medium having stored the program element thereon is provided.

In other words, according to the invention, a reference image or sequence (angiogram, CT or CTA, etc.) either acquired pre-op or at the start of the intervention is used and an anatomical map of the vasculature is made. During the interventional procedure, the interactions and locations, e.g. balloon inflations, stent placement, functional measurements, IVUS, OCT, QCA, are automatically identified and recorded on the live image, either by image detection of the fluoroscopy or image acquisition or by co-registration using other location specific information (e.g. automatic pullback, FFR). Then, this position and action is translated and marked in the reference anatomical map of the object. The reference anatomical map, with the interaction history is then visualized, e.g. on the pre-op angiogram of cardiac roadmap, using several visualization methods, like overlays, colors, pictorials or actual images of the interaction. Next to these interaction visualizations, information can be provided, with characteristics of the interaction, like interaction order/time, functional measurements (FFR/IFR), stent-type etc. The thus created augmented anatomical map is provided during an interventional procedure, e.g. by displaying the augmented anatomical map.

These and other aspects of the present invention will become apparent from and be elucidated with reference to the embodiments described hereinafter.

### BRIEF DESCRIPTION OF THE DRAWINGS

Exemplary embodiments of the invention will be described in the following with reference to the following drawings:
Fig. 1: an embodiment of a device according to the invention;
Fig. 2: an embodiment of a system according to the invention;
Fig. 3: an embodiment of a method according to the invention;
Fig. 4A: a reference anatomical map according to the invention;
Fig. 4B: the reference anatomical map of Fig. 4A;
Fig. 4C: a combination of the reference anatomical map of Fig. 4A and 4B;
Fig. 5A: an event detection according to the invention;
Fig. 5B: the event detection of Fig. 5A;
Fig. 5C: a combination of the event detection of Fig. 5A and 5B;
Fig. 6A: an augmented anatomical map according to the invention;
Fig. 6B: the augmented anatomical map of Fig. 6A;
Fig. 6C: a combination of the augmented anatomical map of Fig. 6A and 6B;

### DETAILED DESCRIPTION OF EMBODIMENTS

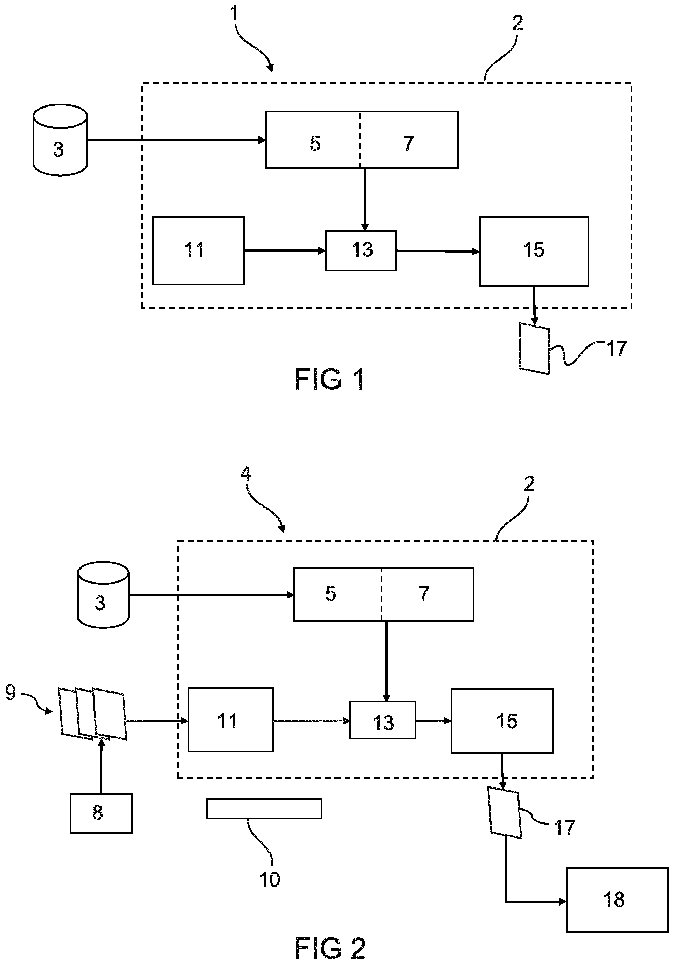

Fig. 1 shows an embodiment of a device 1 for augmenting an anatomical map of an object according to the invention. A processing unit 2 of the device 1 provides a connection to a storage unit 3. One or more reference anatomical maps of the object (not shown) is provided by the storage unit 3. In an embodiment, the reference anatomical map may be a previously generated augmented anatomical map 17 of the object. Additional information of the object, which is a human organ of a patient, for example, such as a human heart, a liver, a brain, arms, legs, etc. can also be provided. The reference anatomical map is a reference image or reference sequence of the object. The reference anatomical map can be acquired pre-operational, i.e. before the actual intervention is performed. In other embodiments, the reference anatomical map can be a previous augmented anatomical map or an atlas, i.e. constructed by combining a large number of anatomies or different patients into a single reference anatomical map. The reference anatomical map could also be acquired at the start of the intervention procedure. Thus, the actual vasculature of the organ along with its events is provided to the processing unit 2.

The processing unit 2 provides a selection unit 5 for selecting the reference anatomical map of the object. In some embodiments, the selection unit also provides a pre-processing unit 7 for pre-processing the selected reference anatomical map.

A detection unit 11 of the processing unit 2 detects one or more events within a space of intervention. The space of intervention is defined according to image data of the object. The event is detected and positioned within the space of intervention. The position of the detected event is translated into the reference anatomical map of the object by a translation unit 13. The event is visualized in the reference anatomical map by a visualization unit 15. The resulting augmented anatomical map 17 of the object is provided by the processing unit 2 during an interventional procedure. The augmented anatomical map provides marked interventional events.

Fig. 2 shows an embodiment of a system 4 for augmenting an anatomical map of an object according to the invention. The system 4 comprises a device 2 (cf. Fig. 1 for description of a device 2). The system 4 comprises an interventional device 10. The interventional device 10 can comprise several interventional imaging systems 10 and is configured for performing balloon inflations, a stent placement, functional measurements, intravascular ultrasound (IVUS), optical coherence tomography (OCT), quantitative coronary angiography (QCA), needle ablation, needle biopsy, beads injection, and the like.

The system 4 comprises an imaging unit 8 for providing a sequence of fluoroscopic images 9. Also, a sequence of angiograms or a sequence of X-ray images can be provided to the processing unit for event detection.

The system 4 further comprises a display unit 18 for displaying the augmented anatomical map 17 of the object during the interventional procedure. The augmented anatomical map 17, which is stored in the storage unit 3, can be displayed (if previously stored) before, during or after the actual interventional procedure, for review or analysis.

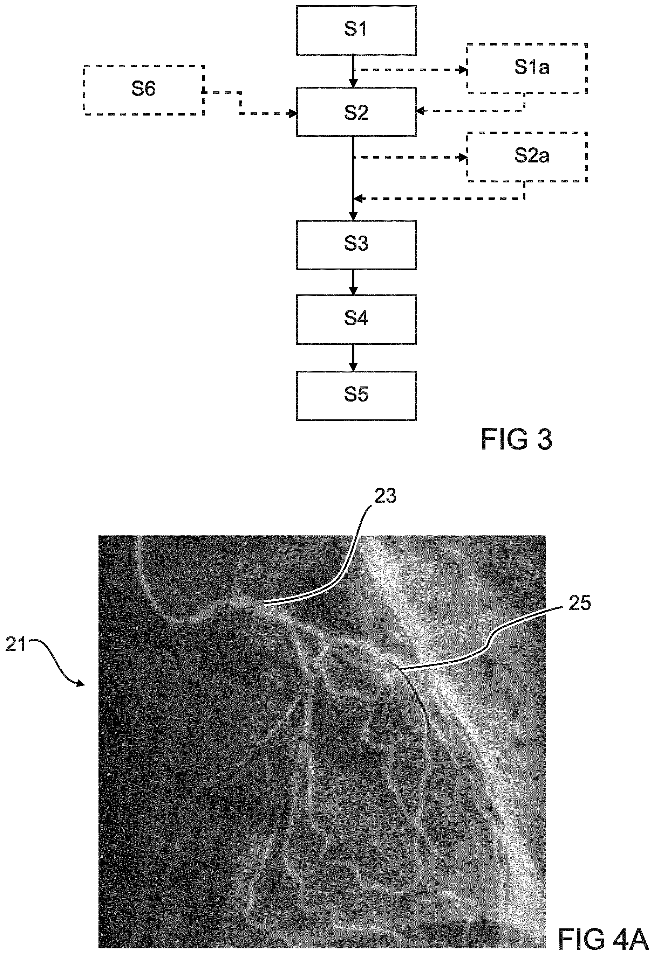

Fig. 3 shows an embodiment of a method for augmenting an anatomical map of an object according to the invention. A practical implementation of the invention can be described as based on four main steps:
Reference anatomical map selection S1 of the object and (optionally) pre-processing S1a of the reference anatomical map;
Live event detection S2;
Translation S3 of the event into the referential of the reference anatomical map;
Visualization S4 and provision S5 of the augmented anatomical map 17 of the object.

According to S1, a reference anatomical map displaying the vessels of the organ of interest is selected. As discussed above, it can be extracted from a pre-intervention exam like a CT, or it can be an injected angiogram from the ongoing examination. The selected reference anatomical map can even be a synthetic map computed from different sources, e.g. different angiograms highlighting different vessels; mixture of CT and angiograms to enrich the 3D content with improved spatial resolution, etc.

The choice of the reference volume(s) and/or image(s) can either be performed by the user, or it can be done automatically by the selection unit 5 of the processing unit 2. The reference anatomical map can either be utilized as such, or it can be pre-processed S1a to offer an improved vessel visibility (background subtraction, volume reprojection from an optimal perspective, etc.).

The event of interest is detected S2 and/or localized in the space of the intervention. One or more events can be also detected automatically S6 in an interventional image of the object.

In this embodiment, the event is detected it in the fluoroscopic images 9 that are acquired during the intervention. For instance, a dilated balloon can be characterized as a regular contrasted region lying between radiopaque markers. The reliable detection of those structures, provides means to detect when a balloon inflation has been performed, and where (in the interventional image) it was deployed. The same approach could be followed to detect OCT, FFRs, IVUS etc. in fluoroscopic images.

Alternatively, one could rely on mechanical measurements performed during certain phases of the intervention. For instance, if an automatic FFR pullback is performed, the exact instant where it begins is known, as well as the pullback velocity. This knowledge, along with event detections in the image (e.g. initial device position), translates in the accurate detection and position of the event over the live intervention image.

After detection of the event of interest, the position of the event is translated S3 into the referential of the chosen reference anatomical map of the object. The event can be an interaction or a location such as balloon inflations, a stent placement, functional measurements, intravascular ultrasound (IVUS), optical coherence tomography (OCT), quantitative coronary angiography (QCA), needle ablation, needle biopsy, beads injection, and the like.

In a specific case, where the selection has been performed over the interventional image, and where the reference anatomical map is an angiogram acquired from the same angulation, this process is known as cardiac roadmapping (see also description if Fig. 4A-C).

For more general situations, the anatomical sub-vessel in which the event has taken place (mid LAD, second diagonal, etc.) could be detected, and translated in the according anatomical position of the reference anatomical map.

Finally, reference anatomical map of the object is augmented by visualizing S4 the event. The augmented anatomical map of the object is provided S5 for further processing and/or display. The augmented anatomical map comprises the information of the reference anatomical map of the object, comprising previous events, and also comprises current live events detected during the interventional procedure. An accurate, detailed and/or precise location of previous or actual live events is made visible and is provided to an interventionist.

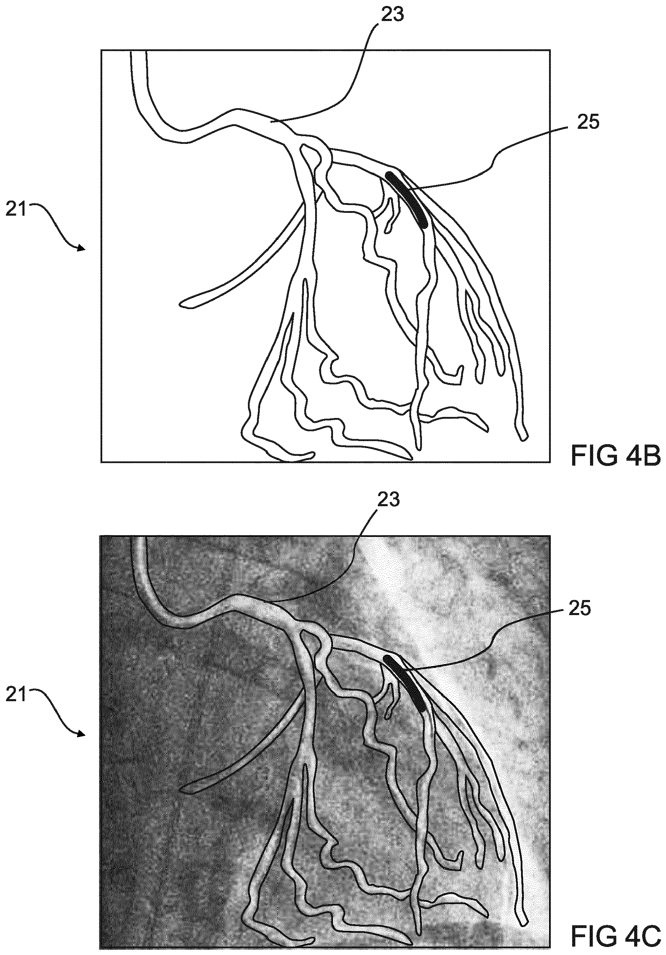

Fig. 4A shows a reference anatomical map 21 according to the invention. The description of the reference anatomical map of Fig. 4A also applies for Fig. 4B and 4C.

In this embodiment, a pre-op angiogram of cardiac roadmap is selected as reference anatomical map 21. Also, other reference anatomical maps can be used, as mentioned above. The vasculature 23 of the object is made visible by the provided cardiac roadmap. An event 25 (for instance a FFR measurement) seems detectable in the reference anatomical map 21.

Fig. 4B shows the reference anatomical map 21 of Fig. 4A wherein the vasculature 23 and other elements are replaced by line drawing for improved visibility.

Fig. 4C shows a combination of the illustration of Fig. 4A and 4B for improved understanding of the provided method and system.

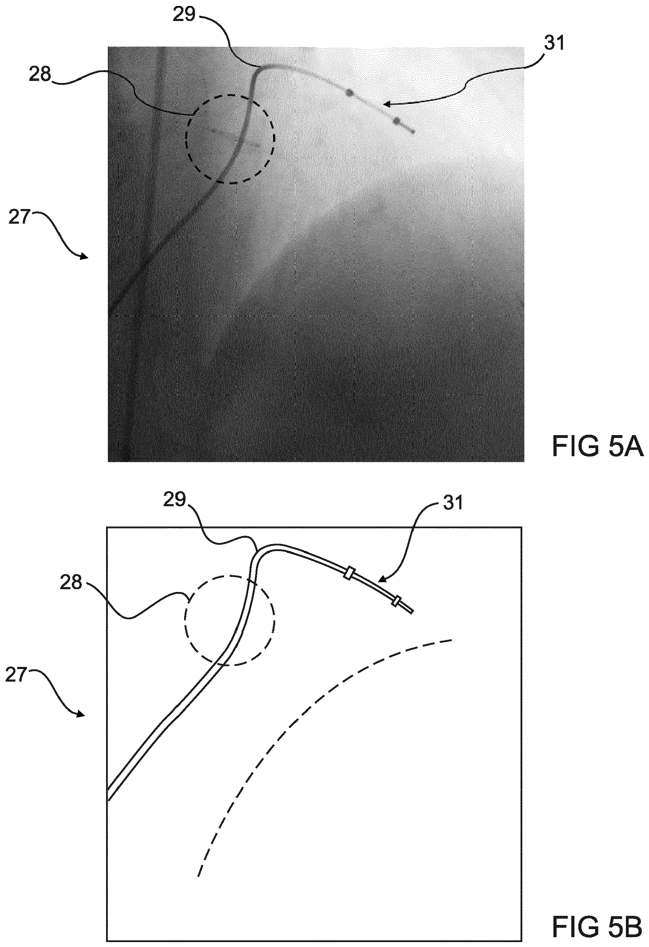

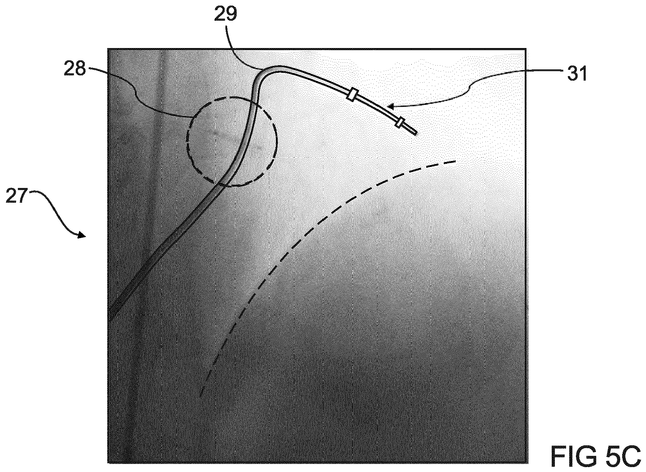

Fig. 5A shows an event 27 according to the invention. The description of the event detection of Fig. 5A also applies for Fig. 5B and 5C.

An event 27 is detected in the fluoroscopic image of Fig. 5A acquired during an intervention. The event 27 is an IVUS intervention and can be detected by detection of a guiding wire 29 of the IVUS during the interventional procedure. However, there is no vasculature detectable in the fluoroscopic image. Region 28, indicated by a dotted circle, might provide for a further event. However, such event is likely to be overseen by an interventionist, as it nearly disappears in the fluoroscopic image of the object.

Detecting those structures of the events provides knowledge of the time the IVUS has been performed, and where it was deployed in the image taken during the interventional procedure.

Fig. 5B shows the event detection 27 of Fig. 5A wherein the guiding wire 29 and other elements are replaced by line drawing for improved visibility.

Fig. 5C shows a combination of the event detection of Fig. 5A and 5B for improved understanding of the provided method and system.

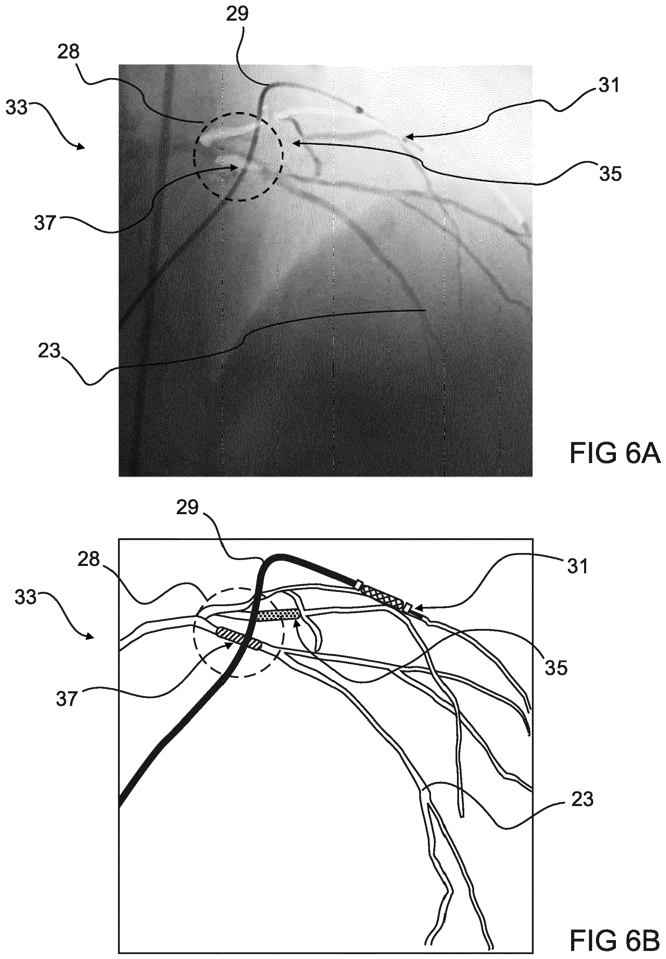

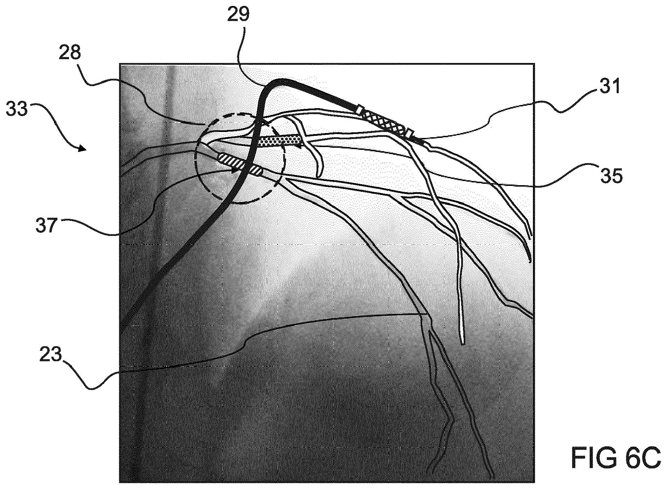

Fig. 6A shows an augmented anatomical map 33 according to the invention. The description of the event detection of Fig. 6A also applies for Fig. 6B and 6C.

The augmented anatomical map 33, with the according interaction history, is visualized on the pre-op angiogram.

Three events 31, 35, 37 have been detected and translated into the selected reference anatomical map 33. Two events 35, 37 have been detected previously and the IVUS interaction (see also Figs. 5A-C) has been detected during an interventional procedure.

The previously detected events 35, 37 are a balloon inflation 35 and a stent 37. The stent 37 is in the region 28 indicated by the dotted circle 28 (not visible in the fluoroscopic image of Figs. 5A-C). In the anatomical map 33 the vasculature 23 of the object is made visible using a unique color, for example. Several visualization methods, like overlays, colors, pictorials or actual images of the interaction can be used to visualize the detected events.

The location of the events 31, 35, 37 that has been determined is highlighted in the augmented anatomical map 33. The events can be marked in reference colors, if the purpose is only to display where some events have taken place. If some measures were made, like FFR for example, these can also be color-coded after translation into the reference anatomical map. The visualization can be marked in reference colors, if the purpose is only to display where some events have taken place. If some measures were made (e.g. FFR), these can also be color-coded (green for high FFR, red for low FFR, for example) when translated into the reference map and made visible in the augmented anatomical map 33 of the object.

Next to these interaction visualizations, information could be presented, with characteristic of the interaction, like interaction order/time, functional measurements (FFR/IFR), stent-type etc.

Fig. 6B shows the augmented anatomical map of Fig. 6A wherein the vasculature 23 and other elements are replaced by line drawing for improved visibility.

Fig. 6C shows a combination of the augmented anatomical map of Fig. 6A and 6B for improved understanding of the provided method and system.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section. A computer program may be stored and/or distributed on a suitable medium, such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated, and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A device of generating an augmented anatomical map of an object, comprising:
- a storage unit, configured for storing a reference anatomical map of the object; and
- a processing unit, configured
- to detect one or more events within a space of intervention, wherein the space of intervention is defined according to image data of the object;
- to translate a position of the event into the reference anatomical map;
- to augment the reference anatomical map by adding information representing the event; and
- to provide the augmented anatomical map of the object;.

2. Device according to claim 1, wherein
- the storage unit is configured for storing multiple reference anatomical maps, and
- the processing unit is configured to select a reference anatomical map of the object from the multiple reference anatomical maps provided by the storage unit.

3. A system of generating an augmented anatomical map of an object, comprising:
- at least one imaging unit;
- a displaying unit; and
- a device according to claim 1;
wherein the augmented anatomical map of the object is provided during an interventional procedure.

4. System according to claim 2, wherein the at least one imaging unit is configured to generate at least an X-ray image.

5. A method of generating an augmented anatomical map of an object, the method comprising the following steps:
detecting one or more events within a space of intervention, wherein the space of intervention is defined according to image data of the object;
translating a position of the event into the reference anatomical map;
augmenting the reference anatomical map by adding information representing the event; and
providing the augmented anatomical map of the object;
wherein the augmented anatomical map of the object is provided during an interventional procedure.

6. Method according to claim 5, wherein the space of intervention comprises the duration of a single intervention or multiple interventions over a period of time.

7. Method according to claim 5 or 6, wherein the reference anatomical map is provided for further processing and/or display.

8. Method according to any of the claims 5 to 7, wherein one or more events are automatically identified in an interventional image of the object.

9. Method according to any of the claims 5 to 8, wherein one or more events are detected in a fluoroscopic image or in a sequence of fluoroscopic images of the object.

10. Method according to any of the claims 5 to 9, wherein measurements performed during the interventional procedure are utilized for detecting the one or more events.

11. Method according to any of the claims 5 to 10, wherein additional information or image processing techniques are used for aiding the detecting of the one or more events, or for calculating the location on the reference anatomical map.

12. Method according to any of the claims 5 to 11, wherein translating the one or more events involves a cardiac roadmapping technique.

13. Method according to any of the claims 5 to 12, wherein the anatomical map is updated using new information acquired during the space of the intervention.

14. A computer program element for controlling an apparatus according to one of the claims 1 to 4, which, when being executed by a processing unit, is adapted to perform the method steps of one of the claims 5 to 13.

15. A computer readable medium having stored the program element of claim 14.
